# EUROPÄISCHE PATENTANMELDUNG

(11) **EP 2 253 281 A2**
(43) Veröffentlichungstag der Anmeldung: **24.11.2010**
(21) Anmeldenummer: 10450082.2
(22) Anmeldetag: 12.05.2010
(51) Int. Cl.: A61B 17/29

(54) **Greifinstrument mit an einem Instrumentenkopf angeordneten Greifbacken**

(30) Priorität: 14.05.2009 AT 7452009
(71) Anmelder: Metzger, Peter, 1210 Wien (AT)
(72) Erfinder: Metzger, Peter, Univ. Doz. Dr. med., 1210 Wien (AT); Matzner, Andreas, Prof. Dipl.-Ing. Dr., 1110 Wien (AT)
(74) Vertreter: Rippel, Andreas

(57) **Zusammenfassung**

Bei einem Greifinstrument mit an einem Instrumentenkopf (6) angeordneten Greifbacken (3) ist der Instrumentenkopf (6) mittels eines Gelenkstifts (7) an einem Rohr (11) schwenkbar gelagert, in dem eine Stange (10) zur Betätigung der Greifbacken (3) geführt ist.

Die Stange (10) ist im Bereich des Gelenkstiftes (7) durch ein elastisches Umlenkelement (13) mit einer auf die Greifbacken (3) wirkenden Betätigungsstange (14) verbunden.

Dadurch behalten die Greifbacken (3) bei Verschwenkung des Instrumentenkopfes (6) ihre jeweilige Stellung.

## Beschreibung

Die Erfindung bezieht sich auf ein Greifinstrument mit an einem Instrumentenkopf angeordneten Greifbacken wobei der Instrumentenkopf mittels eines Gelenkstifts an einem Rohr schwenkbar gelagert ist, in dem eine Stange zur Betätigung der Greifbacken geführt ist. Insbesondere soll das erfindungsgemäße Greifinstrument in der "minimal invasiven Chirurgie" Anwendung finden.

Die minimal-invasive Chirurgie (MIC), bekannt auch als Schlüssellochchirurgie, Knopflochchirurgie, Laparoskopie im Bauchbereich, Thoraskopie im Brustkörper, wird angewendet für operative Eingriffe mit kleinst möglicher Verletzung von Haut und Weichteilen, wobei der Chirurg durch einen schmalen Schnitt, eine Inzision und/oder eine natürliche Körperöffnung das chirurgische Instrument in den Körper an sein Ziel führt.

Die Vorteile der minimal invasiven Operationstechnik ergeben sich einerseits durch die viel kleineren Schnitte ("Inzisionen"), die die Bauchdecke bzw. den Brustkorb weitgehend unversehrt lassen, andererseits treten bei dieser Operationstechnik keine physikalischen Belastungen (Zug, Druck, etc.) für Bauchwand, Bauchfell ("Peritoneum") und benachbarte Organe durch die Verwendung von Haken, Bauchtüchern, Spateln etc., auf. Daher hat der Patient nach dem Eingriff wesentlich weniger Schmerzen, benötigt keine oder nur wenig Schmerzmittel und kann sehr bald nach der Operation normal atmen, husten und aufstehen.

Betriebswirtschaftlich betrachtet sind zwar höhere Operationskosten (Instrumente, Technologie wie Videokamera und -Monitor, Apparate für die Gasinsuffation etc.), teilweise aufwendigere Narkoseverfahren und in der Anfangsphase längere Operationszeiten zu berücksichtigen, dem gegenüber sinken jedoch die Dauer des Krankenhausaufenthaltes und die Komplikationsraten.

Volkswirtschaftlich gesehen wirken sich die signifikante Verkürzung sowohl des Krankenhausaufenthaltes als auch der Rekonvaleszenzzeit sowie der Arbeitsunfähigkeit positiv aus.

Die Anwendung in der MIC stellt an chirurgische Instrumente sehr hohe Anforderungen an ihre Funktionalität und Handhabung. Einerseits müssen sie eine entsprechende Steifigkeit aufweisen, aber andererseits sollten sie entsprechende Flexibilität und Elastizität besitzen, um problemlos und schnell einen Eingriff zu bewältigen.

Alle herkömmlichen chirurgischen Greifinstrumente erweisen sich aufgrund ihrer aufwendigen Handhabung und mangelhaften Flexibilität des Greifens, nur bedingt für präzise chirurgische Eingriffe geeignet, da sie nur eine Drehbewegung ausführen und ihre Greifbacken nur in Längsrichtung greifen können.

Ziel vorliegender Erfindung ist es, bei einem Greifinstrument mit einem gelenkig verbundenen Instrumentenkopf zu erreichen, dass bei Verschwenkung des Instrumentenkopfes die an ihm angeordneten Greifbacken ihre jeweilige Stellung behalten. Sind die Greifbacken geschlossen, dann sollen sie auch bei einer Verschwenkung des Instrumentenkopfes geschlossen bleiben.

Dies wird bei einem erfindungsgemäßen Greifinstrument der eingangs genannten Art dadurch erreicht, dass die Stange im Bereich des Gelenkstiftes durch ein elastisches Umlenkelement mit einer auf die Greifbacken wirkenden Betätigungsstange verbunden ist.

Durch diese erfindungsgemäße Maßnahme wird bei Auslenkung des Instrumentenkopfes keine Bewegung der Greifbacken hervorgerufen.

Bei einer vorteilhaften Ausführungsform der Erfindung ist das elastische Umlenkelement in einem Schlitz im Gelenkstift geführt.

Nachstehend ist die Erfindung anhand eines in den Zeichnungen dargestellten Ausführungsbeispieles, auf das die Erfindung jedoch keineswegs beschränkt ist, näher beschrieben.

Dabei zeigen:
Fig. 1 in schaubildlicher Ansicht ein erfindungsgemäßes Greifinstrument, wobei die Greifbacken geöffnet sind;
Fig. 2 ebenfalls in schaubildlicher Ansicht den Unterteil des erfindungsgemäßen Greifinstruments in einer gegenüber der Fig. 1 vergrößerten Darstellung und mit geschlossenen Greifbacken;
Fig. 3 eine der Fig. 2 entsprechende Darstellung, aber mit geöffneten Greifbacken;
Fig. 4 ein Detail in vergrößertem Maßstab.

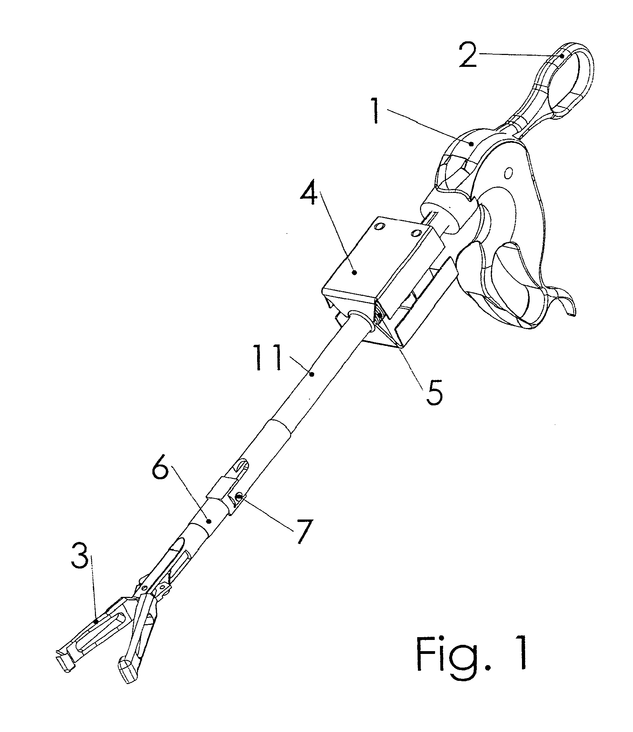

Fig. 1 zeigt das erfindungsgemäße flexible chirurgische Greifinstrument mit Handgriff 1, dem Betätigungshebel 2 zum Öffnen und Schließen der Greifbacken 3, den Rasterbacken 4 mit der Rasterung 5, die den Instrumentenkopf 6 mit den Greifbacken 3 in ausgelenkter Position halten. Um die Achse 7 kann der Instrumentenkopf 6 mit den Greifbacken 3 aus seiner geradlinigen Position ausgelenkt werden.

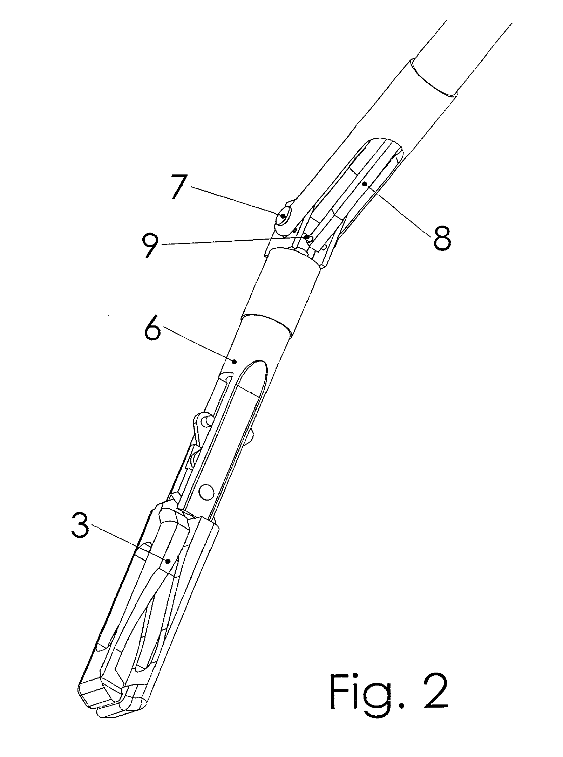

Fig. 2 zeigt die Auslenkung des Instrumentenkopfs 6, wobei die Greifbacken 3 geschlossen sind. Diese Auslenkung erfolgt durch eine Schubstange 8, die mit den Rasterbacken 4 verbunden ist. Die Schubstange 8 greift auf einen Stift 9, der mit dem Instrumentenkopf 6 verbunden ist, wobei der Stift 9 nicht konzentrisch zur Achse 7 liegt. Die Längsbewegung der Schubstange 8 erfolgt durch die manuelle Verschiebung der Rasterbacken 4. Durch Längsbewegung der Schubstange 8 wird der Instrumentenkopf 6 mit den Greifbacken 3 aus seiner geradlinigen Position ausgelenkt.

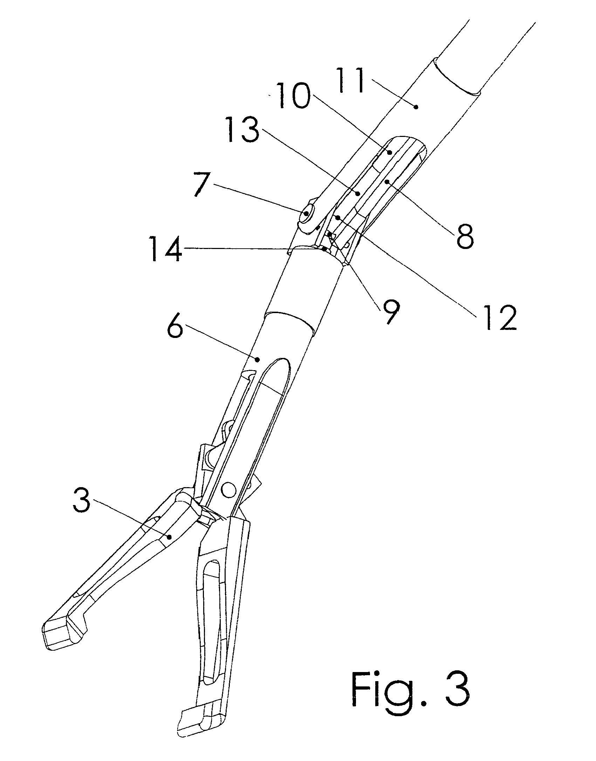

Fig. 3 zeigt die Auslenkung des Instrumentenkopfs 6, wobei die Greifbacken 3 geöffnet sind. Durch Betätigung des Betätigungshebels 2 verschiebt sich die geteilte Stange 10, welche im Hauptrohr 11 gelagert ist und über ein durch einen Schlitz 12 in der Achse 7 durchgeführtes Umlenkelement 13 mit der Betätigungsstange 14 verbunden ist. Durch diese Betätigungsstange 14 werden die Greifbacken 3 geöffnet oder geschlossen.

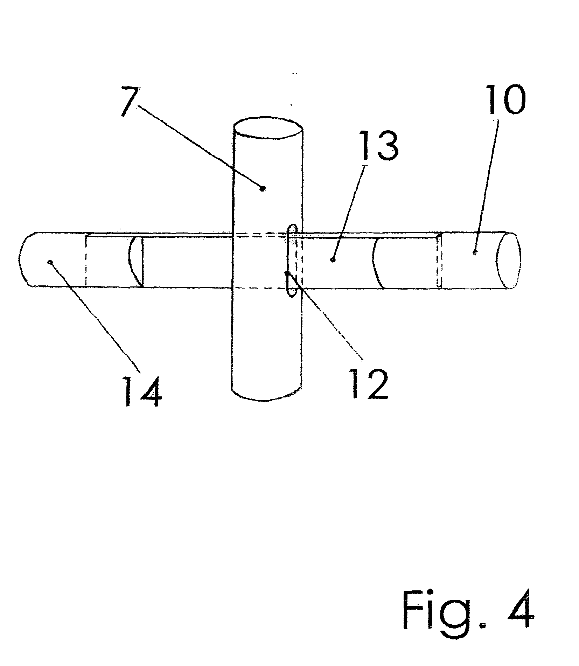

Insbesondere aus Fig. 4 ist eine vorteilhafte Weiterbildung der beschriebenen Anordnung ersichtlich, wonach das Umlenkelement 13 zwischen der Stange 10 und der Betätigungsstange 14 als biegsames Element ausgeführt und zentrisch durch einen Schlitz 12 in der Achse 7 durchgeführt ist. Dadurch wird bei Auslenkung des Instrumentenkopfs 6 keine Bewegung der Greifbacken 3 verursacht.

Im Rahmen der Erfindung sind zahlreiche Abänderungen möglich. Insbesondere ist die Erfindung nicht auf chirurgische Instrumente beschränkt. Ihre Anwendung kann vielmehr in allen jenen Fällen erfolgen, bei denen nur durch eine kleine Öffnung hindurch Handlungen durchzuführen sind. Erwähnt können auch diagnostische oder therapeutische Handlungen werden.

## Patentansprüche

1. Greifinstrument mit an einem Instrumentenkopf (6) angeordneten Greifbacken (3), wobei der Instrumentenkopf (6) mittels eines Gelenkstifts (7) an einem Rohr (11) schwenkbar gelagert ist, in dem eine Stange (10) zur Betätigung der Greifbacken (3) geführt ist, **dadurch gekennzeichnet, dass** die Stange (10) im Bereich des Gelenkstiftes (7) durch ein elastisches Umlenkelement (13) mit einer auf die Greifbacken (3) wirkenden Betätigungsstange (14) verbunden ist.

2. Greifinstrument nach Anspruch 1, **dadurch gekennzeichnet, dass** das elastische Umlenkelement (13) in einem Schlitz (12) im Gelenkstift (7) geführt ist.
